# EUROPEAN PATENT SPECIFICATION

(11) **EP 3 386 942 B1**
(45) Date of publication and mention of the grant of the patent: **18.09.2019**
(21) Application number: 16806186.9
(22) Date of filing: 09.12.2016
(51) Int. Cl.: C07C 203/00, C07F 15/02, C07F 15/04, C07F 15/06

(54) **METHOD FOR PREPARING SALTS OF ARENE-TRANSITION METAL SANDWICH COMPLEXES**
VERFAHREN ZUR HERSTELLUNG VON SALZEN AUS AREN-ÜBERGANGSMETALL-SANDWICHKOMPLEXEN
PROCÉDÉ DE PRÉPARATION DE SELS DE COMPLEXES SANDWICH ARÈNE-MÉTAL DE TRANSITION

(30) Priority: 10.12.2015 EP 15199397
(43) Date of publication of application: 17.10.2018
(73) Proprietor: BASF SE, 67056 Ludwigshafen am Rhein (DE)
(72) Inventor: HEMGESBERG, Maximilian, 67663 Kaiserslautern (DE); FRANK, Jürgen, 67065 Ludwigshafen (DE); NÖRENBERG, Ralf, 69123 Heidelberg (DE)
(74) Representative: BASF IP Association
(86) International application number: PCT/EP2016/080410
(87) International publication number: WO 2017/097969

(56) References cited:
- M. LE RUDULIER ET AL: "Préparation de cations [[eta]5-cyclopentadiényl fer [eta]6-arène]+ comportant une fonction oxime, cétone, amine ou alcool en [alpha] du ligande arène", JOURNAL OF ORGANOMETALLIC CHEMISTRY., vol. 310, no. 2, 1 August 1986 (1986-08-01), pages 209-224, XP055334630, CH ISSN: 0022-328X, DOI: 10.1016/S0022-328X(00)99554-8
- YU-PIN WANG ET AL: "Synthesis of (Cl)(NO)2Cr(n5-C5H4)C(O)(n5-C5H4)Fe-(n5-C5 H5). An unexpected product from the Friedel-Crafts acylation of (CO)2(NO)Cr(n5-C5H4)C(O)(n5-C5H4-Fe(n5-C5H 5) with (CO)2(NO)Cr[(n5-C5H4)C(O)Cl]", JOURNAL OF ORGANOMETALLIC CHEMISTRY., vol. 399, 1990, pages 141-147, XP002765913, ELSEVIER-SEQUOIA S.A. LAUSANNE. ISSN: 0022-328X
- P. ZANELLO ET AL.: "The redox behaviour of ferrocene derivatives: VI. Benzylferrocenes. The crystal structure of decabenzylferrocenium tetrafluoroborate", JOURNAL OF ORGANOMETALLIC CHEMISTRY, vol. 471, no. 1-2, 31 May 1994 (1994-05-31), pages 171-177, XP002765914, ISSN: 0022-328X cited in the application

## Description

The present application relates to a method for preparing a salt consisting of cations of an arene-transition metal sandwich complex and anions of a Brönsted acid. Said salts are herein also referred to as salts of arene-transition metal sandwich complexes.

Certain metallocenium salts, for instance bis(cyclopentadienyl)ferrocenium hexafluorophosphate, are obtainable by a two-step method comprising oxidation of the corresponding metallocene, e.g. ferrocene, with concentrated sulfuric acid to the corresponding metallocenium cation, and subsequent metathesis with an alkali salt having the desired anion, e.g. NaPF₆ (see E.S. Yang et al., The Journal of Physical Chemistry, Vol. 79, No. 19, 1975).

Alternatively, ferrocenium salts are obtainable by oxidizing the corresponding ferrocene with a silver salt of the desired anion or with a nitrosyl salt of the desired anion. For instance, decabenzyl ferrocenium tetrafluoroborate is obtainable by oxidizing decabenzyl ferrocene with NOBF₄ in CH₂Cl₂ (see P. Zanello et al., Journal of Organometallic Chemistry, 471 (1994) 171-177 171).

However, for certain anions it is difficult to prepare a nitrosyl salt. For instance, following the teaching of P. Zanello et al., ferrocenium salts of bis(trifluoromethyl-sulfonyl)imide could be obtained by oxidizing the corresponding ferrocene with the nitrosyl salt of bis(trifluoromethylsulfonyl)imide ((CF₃SO₂)₂NNO). However, (CF₃SO₂)₂NNO is obtainable only by means of a complex multistage synthesis (see J. Ferropoulos et al., Inorg. Chem. 1984, 23, 3720-3723).

Accordingly, for preparing salts of arene-transition metal sandwich complexes, especially ferroceniums salts like ferrocenium bis(trifluoromethyl-sulfonyl)imide, a method is needed which is less complex and uses a readily available oxidizing agent.

Surprisingly it has been found that said salts are obtainable in a single pot reaction by oxidizing an arene-transition metal sandwich complex, e.g. a ferrocene, in the presence of a Brönsted acid comprising the desired anion using an alkyl nitrite as the oxidizing agent.

Accordingly, in a first aspect the present invention relates to a method for preparing a salt consisting of cations of an arene-transition metal sandwich complex (i) and anions of a Brönsted acid (iii)
said method comprising
reacting a reaction mixture comprising, dissolved in a solvent, the reactants
(i) an arene-transition metal sandwich complex selected from the group consisting of bis-benzenechromium and metallocenes
(ii) an oxidizing agent selected from the group consisting of alkyl nitrites
(iii) a selected Brönsted acid.

Arene-transition metal sandwich complexes (i) are complexes comprising a transition metal central atom which is coordinatively linked to the π-electrons systems of two aromatic ligands which surround said transition metal central atom in a sandwich-like structure, i.e. the transition metal central atom is located between the faces of two parallel aromatic ligands. The two ligands of said sandwich complex may be the same (homoleptic sandwich complex) or different (heteroleptic sandwich complex).

The aromatic ligands are selected from the group consisting of benzenoid aromatic ligands (e.g. benzene) and non-benzenoid aromatic ligands (e.g. cyclopentadienyl). An exemplary arene-transition metal sandwich complex wherein the two aromatic ligands are benzenoid is bis-benzenechromium wherein a chromium central atom is situated between two benzene ligands in a sandwich-like structure. Other arene-transition metal sandwich complexes wherein the two aromatic ligands are benzenoid are known to the skilled person. Exemplary arene-transition metal sandwich complexes wherein the two aromatic ligands are non-benzenoid are metallocenes.

Said arene-transition metal sandwich complex (i) is selected from the group consisting of bis-benzenechromium and metallocenes. Preferably, said arene-transition metal sandwich complex (i) is a metallocene selected from the group consisting of ferrocenes, nickelocenes, cobaltocenes and osmocenes.

Metallocenes are bis(η⁵-cyclopentadienyl) complexes of transition metals. In said complexes, the transition metal central atom is situated between two cyclopentadienyl ligands in a sandwich-like structure. The transition metal is preferably selected from the group consisting of iron, nickel, cobalt and osmium. The corresponding metallocene complexes are referred to as ferrocenes, nickelocenes, cobaltocenes and osmocenes, resp.. Other metallocenes are known to the skilled person.

Arene-transition metal sandwich complexes (i) such as metallocenes are electrically neutral molecules. As known by the skilled person, in metallocenes the η⁵⁻cyclopentadienyl ligand is in the form of an anion (cyclopentadienyl anion), also referred to as cyclopentadienide.

In a metallocene complex, the transition metal central atom is coordinatively linked to the π-electrons system of each of the two η⁵-cyclopentadienyl ligands. Herein the term cyclopentadienyl ligand includes cyclopentadienyl ligands wherein each carbon atom of the cyclopentadienyl ring is linked to a hydrogen atom [η⁵-C₅H₅] as well as those wherein one, more or all carbon atoms of the cyclopentadienyl ring are linked to a substituent (residue) R' different from hydrogen ([η⁵-C₅R'ₘH₅₋ₘ] wherein 0 < m ≤ 5), wherein each R' is independently selected from the group consisting silyl residues, alkyl residues and aralkyl resides. The term "alkyl" herein refers to the radical of saturated aliphatic groups, including linear-chain alkyl groups, branched-chain alkyl groups, cycloalkyl (alicyclic) groups, alkyl substituted cycloalkyl groups, and cycloalkyl substituted alkyl groups. Preferred alkyl residues are methyl, ethyl, and iso-propyl. The term "aralkyl" herein refers to aryl-substituted alkyls, e.g. benzyl -CH₂-C₆H₅ or phenylethyl -CH₂-CH₂C₆H₅.

Metallocenes having cyclopentadienyl ligands [η⁵-C₅H₅] wherein each carbon atom of the cyclopentadienyl ring is linked to a hydrogen atom are hereinbelow referred to as unsubstituted metallocenes. Metallocenes having cyclopentadienyl ligands ([η⁵-C₅R'ₘH₅₋ₘ] wherein 0 < m ≤ 5) wherein one, more or all carbon atoms of the cyclopentadienyl ring are linked to a substituent (residue) R' different from hydrogen are hereinbelow referred to as substituted metallocenes. Typically in substituted metallocenes each carbon atom of the two cyclopentadienyl rings is linked to a substituent (residue) R' selected from the group consisting of silyl residues, alkyl residues and aralkyl residues, so that the cyclopentadienyl ligands have the structure [η⁵-C₅R'₅]. In certain cases it is preferred that the ten residues R' of such metallocene complex have the same chemical structure, preferably methyl -CH₃ or benzyl -CH₂-C₆H₅. Examples of such substituted metallocenes are decamethyl ferrocene, decabenzyl ferrocene, decamethyl osmocene and decamethyl cobaltocene. Other substituted metallocenes are known to the skilled person. The term metallocenes as used herein includes both unsubstituted metallocenes as defined above and substituted metallocenes as defined above.

Moreover, in a substituted metallocene the η⁵-cyclopentadienyl ligand may be in the form of a polycyclic species comprising an anionic cyclopentadienyl ring and annulated to said cyclopentadienyl ring one or two further rings, e.g. benzene rings, to which further rings, e.g. benzene rings, may be annulated. Those η⁵-cyclopentadienyl ligands having one or more benzene rings annulated to the cyclopentadienyl ring are also referred to as benzannulated cyclopentadienides. A cyclopentadienyl ligand consisting of a cyclopentydienyl ring and a benzene ring annulated to said cyclopentadienyl ring is referred to as an indenyl ligand or indenide ligand. A cyclopentadienyl ligand consisting of a cyclopentydienyl ring and two benzene rings each annulated to said cyclopentadienyl ring is referred to as a fluorenide ligand (derived from fluorene). Annulation of a further benzene ring to one resp. both of the benzene rings of the fluorenide ligand results in a benzofluorenide ligand resp, dibenzofluorenide ligand (derived from benzofluorene resp. dibenzofluorene). Those ligands and corresponding metallocenes are described e.g. in F. Pammer, Y. Sun, C. May, G. Wolmershäuser, H. Kelm, H.-J. Krüger and W. R. Thiel, Angew. Chem. Int. Ed. 2007, 46, 1270-1273, and Frank Pammer, Yu Sun, Markus Pagels, Daniel Weismann, Helmut Sitzmann and Werner R. Thiel, Angew. Chem. Int. Ed. 2008, 47, 3271 -3274. A η⁵-cyctopentadienyl ligand derived from 5H-dibenzo[e,h]-dibenzo[3,4:6,7]cyclohept[1,2-a]azulene is disclosed in Organometallics 2015, 34, 5374-5382.

Furthermore, a substituted metallocene may be in the form of a metallocenophane (also referred to as an ansa-metallocene). Metallocenophanes are metallocenes which contain a bridging moiety between the two η⁵-coordinated cyclopentadienyl rings. Two main classes of metallocenophanes are known: in [m]metallocenophanes the ligands of one metallocene are connected by a bridge (or by several bridges); in [m.n]metallocenophanes two (or more) metallocenes are brought together into one molecule by bridging groups, see U. T. Mueller-Westerhoff, Angew. Chem. Int. Ed. Engl. 25 (1986) 702-717. For instance a species containing two bridged η⁵cyclopentadienyl rings is in the form of a cyclopentadienyl ring carrying an aralkyl substituent which in turn carries another cyclopentanyl ring. Examples of metallocenophanes are known, e.g. from Angewandte Chemie, volume 98, no. 8, 1986, pages 700-716.

In particularly preferred methods according to the present invention, said metallocene is selected from the group consisting of decamethyl ferrocene, 1,1',3,3',4,4'-hexaisopropyl-2,2',5,5'-tetramethyl ferrocene, 3,3',4,4'-tetraisopropyl-1,1',2,2',5,5'-hexamethyl ferrocene, 1,1'-diethyl-2,2',5,5'-tetramethyl-3,3',4,4'-tetraisopropyl ferrocene, 1,1'-bis(trimethylsilyl)-2,2',5,5'-tetramethyl-3,3',4,4'-tetraisopropyl ferrocene and octaisopropyl ferrocene.

Arene-transition metal sandwich complexes (i) are electrically neutral molecules. An arene-transition metal sandwich complex can be oxidized into a cation, herein referred to as the cation of said arene-transition metal sandwich complex. In the method according to the present invention, an arene-transition metal sandwich complex (i) is oxidized into the corresponding cation by means of an oxidizing agent (ii) selected from the group consisting of alkyl nitrites.

Alkyl nitrites (ii) are also referred to as nitrous acid esters. Alkyl nitrites have a structure according to the general formula R-O-N=O, wherein R is an alkyl. The term "alkyl" refers to the radical of saturated aliphatic groups, including linear-chain alkyl groups, branched-chain alkyl groups, cycloalkyl (alicyclic) groups, alkyl substituted cycloalkyl groups, and cycloalkyl substituted alkyl groups.

Typical alkyls R are methyl, ethyl, 1-propyl, 2-propyl, 1-butyl, 2-butyl, 1-pentyl, 2-pentyl, 3-pentyl, 1-hexyl, 2-hexyl, 3-hexyl, 2-methylprop-1-yl, 2-methylprop-2-yl, 2-methylbut-1-yl, 3-methylbut-1-yl (also referred to as isoamyl), 2-methylbut-2-yl, 3-methylbut-2-yl, 2-methylpent-1-yl, 2-methylpent-2-yl, 2-methylpent-3-yl, 3-methylpent-1-yl, 3-methylpent-2-yl, 3-methylpent-3-yl, 4-methylpent-1-yl and 4-methylpent-2-yl.

Alkyl nitrites are generally known in the field of organic synthesis, because they are commonly used as nitrosation agents in nitrosation reactions. Alkyl nitrites are commercially available. Preferably, in the method according to the present invention the alkyl nitrite (ii) is isoamyl nitrite, because it is readily available.

Without wishing to be bound by any theory, it is presently assumed that when the reaction mixture is allowed to react, molecules of alkyl nitrite (ii) R-O-N=O decompose into
- a nitrosyl cation NO⁺ which oxidizes the arene-transition metal sandwich complex into the corresponding cation while the nitrosyl cation is reduced to gaseous oxides of nitrogen, especially nitric oxide NO, and
- an alcoholate anion RO⁻ which accepts H⁺ released from the Brönsted acid (iii), thus forming the corresponding alcohol ROH.

In the method according to the present invention, said arene-transition metal sandwich complex (i) is oxidized in the presence of a selected Brönsted acid (iii) comprising the anion of the salt to be produced. Brönsted acids (iii) are proton donators, i.e. compounds capable of donating protons to a compound which is a proton acceptor, i.e. capable of accepting the donated protons (Brönsted base). When donating a proton, the Brönsted acid is transformed into its corresponding base.

Said Brönsted acid (iii) is typically an electrically neutral compound of the general formula HX wherein H corresponds to an ionogenic proton and X corresponds to an acid residue anion. The anion X⁻ formed when the ionogenic proton is released from the Brönsted acid (iii) is the corresponding base of said non-aqueous Brönsted acid HX. It is herein referred to as the anion of said Brönsted acid (iii). Said anion of said Brönsted acid (iii) is the anion of the salt to be formed by the method of the present invention.

In the method according to the present invention the Brönsted acid (iii) is selected to meet the following requirements:
- it is not oxidized by the oxidizing agent (ii) selected from the group consisting of alkyl nitrites
- it does not act as an oxidizing agent with respect to the arene-transition-metal sandwich complex (i) of which a salt is to be prepared.

Said Brönsted acid (iii) is not selected from the group consisting of nitric acid, perchloric acid and sulfuric acid.

Preferably said Brönsted acid (iii) is a sulfonylimide R¹-SO₂NH-SO₂-R² wherein R¹ and R² are independently selected from the group consisting of CF₃, CHF₂ and CH₂F. Further preferably, said Brönsted acid (iii) is bis(trifluoromethylsulfonyl)imide (CF₃SO₂)₂NH.

Other preferred Brönsted acids (iii) are fluoroboric acid HBF₄, hexafluorophosphoric acid HPF₆ and trifluoroacetic acid. Still other preferred Brönsted acids (iii) are selected from the group of fluorinated alcohols and fluorinated phenols which are capable of releasing an ionogenic proton. Herein, hexafluoro-isopropanol HO-CH(CF₃)₂, perflorinated alcohols, e.g. perfluoro-tert-butyl alcohol HO-C(CF₃)₃ and perfluoro methanol HO-CF₃, and perfluorinated phenols, e.g. perfluoro phenol HO-C₆F₅, are particularly preferred.

In the reaction mixture, said oxidizing agent (ii) oxidizes the arene-transition metal sandwich complex (i) into the corresponding cations, and said Brönsted acid (iii) is the source of anions compensating the charge of the cations obtained by oxidizing said arene-transition metal sandwich complex.

Preferably, the reaction mixture is prepared by dissolving in a solvent
(i) an arene-transition metal sandwich complex selected from the group consisting of bis.benzenechromium and metallocenes
(ii) an oxidizing agent selected from the group consisting of alkyl nitrites
(iii) a selected Brönsted acid.

Preferably, each of reactants (i), (ii) and (ii) is dissolved in the solvent in a concentration of from 0.05 mol/L to 1.5 mol/L, preferably of from 0.075 mol/L to 1.0 mol/L.

The solvent is selected to meet the following requirements:
- capability of dissolving the above-defined reactants (i), (ii) and (iii) so as to form a reaction mixture
- undergoing no chemical reaction with reactants (i), (ii) and (iii)
- low dissolving capability for ionogenic compounds like salts, so that the reaction product which is a salt precipitates and is readily separated from the reaction mixture.

Preferably, the dissolving capability of the solvent for the reaction product (salt of arene-transition metal sandwich complex) is below 0.05 mol/L, preferably below 0.01 ml/L, further preferably below 0.001 mol/L.

These requirements are fulfilled by solvents selected from the group of aprotic solvents. Aprotic solvents are solvents which do not contain ionogenic protons in their molecules.

Preferably, the solvent for preparing the reaction mixture by dissolving components (i), (ii) and (iii) as defined above is selected from the group consisting of chlorinated hydrocarbons, toluene, diethyl ether, methyl-tert-butyl-ether, tetrahydrofurane and esters of carboxylic acids. Preferred esters of carboxylic acids are selected from the group consisting of ethyl acetate, n-propyl acetate, i-propyl acetate, n-butyl acetate and i-butyl acetate. Preferred chlorinated hydrocarbons are selected from the group consisting of CH₂Cl₂, CHCl₃ and CCl₄.

Preferably,
(i) said arene-transition metal sandwich complex and
(ii) said oxidizing agent
are dissolved in the solvent in a molar ratio (i)/(ii) in the range of from 0.8:1 to 1:0.8, preferably 0:9:1 to 1:0.9, further preferably 0.95:1 to 1:0.95.

In some cases it is preferred that proceeding of the reaction is enhanced by heating the reaction mixture to its boiling point under reflux conditions. Herein, preferably, reacting the reaction mixture comprises keeping the reaction mixture at its boiling point under reflux conditions. Preferably the reaction mixture is kept at its boiling point under reflux conditions for a duration of at most two hours, preferably for less than two hours, e.g. one hour or less. In other cases, it is preferred to let the reaction mixture react at room temperature.

Surprisingly it has been found that the reaction in the reaction mixture starts and proceeds very fast as can be recognized by a color change of the reaction mixture (for instance the reaction mixture achieves a dark green coloration in the case of forming decamethyl ferrocenium salts like decamethyl ferrocenium bis(trifluoromethylsulfonyl) imide) and gas evolution. Due to the release of gaseous oxides of nitrogen, the reaction is irreversible.

Preferably, a method according to the present invention further comprises the steps of
- evaporating the solvent from the reaction mixture to obtain a solid residue comprising the reaction product (salt of arene-transition metal sandwich complex)
- digesting the obtained solid residue with a digesting agent
- removing the digesting agent from the digested solid residue
- drying the digested solid residue.

Evaporating the solvent from the reaction mixture is preferably carried out by means of applying a pressure below pₙ = 101 325 Pa.

The digesting agent used in the step of digesting the obtained solid residue is selected to have a low dissolving capability for ionogenic compounds (salts) so that only negligible amounts of the reaction product which is a salt dissolve in the step of digesting. Preferably, the digesting agent has a comparably low or even lower dissolving capability for the reaction product than the solvent of the reaction mixture. These requirements are fulfilled by digesting agents which are aprotic, i.e. do not contain ionogenic protons in their molecules.

In said step of digesting the obtained solid residue, said digesting agent may be selected from the same group as the solvent used for preparing the reaction mixture. Preferably, in said step of digesting the obtained solid residue, said digesting agent is selected from the group consisting of chlorinated hydrocarbons, toluene, diethyl ether, methyl-tert-butyl-ether, tetrahydrofurane and esters of carboxylic acids. Preferred chlorinated hydrocarbons are selected from the group consisting of CH₂Cl₂, CHCl₃ and CCl₄. Preferred esters of carboxylic acids are selected from the group consisting of ethyl acetate, n-propyl acetate, i-propyl acetate, n-butyl acetate and i-butyl acetate.

For instance, the solvent used for preparing the reaction mixture is selected from the group consisting of CH₂Cl₂, CHCl₃ and CCl₄, and the digesting agent used for digesting the obtained solid residue is diethyl ether or toluene.

In other preferred methods according to the present invention, the solvent used for preparing the reaction mixture and the digesting agent used for digesting the obtained solid residue have the same chemical composition.

Removing the digesting agent from the digested solid residue is preferably achieved by filtration.

Drying of the digested solid residue is preferably carried out in a temperature range of from 0 °C to 100 °C, preferably of from 20 °C to 50 °C. Preferably, in the step of drying, the digested solid residue is subject to a pressure below pₙ = 101 325 Pa.

In preferred methods according to the present invention, a premixture comprising
(i) said arene-transition metal sandwich complex
(ii) said alkyl nitrite
is prepared first by dissolving (i) said arene-transition metal sandwich complex and (ii) said alkyl nitrite in a solvent, and then said Brönsted acid (iii) is added to said premixture,

thus obtaining the reaction mixture. Preferably, a solution of said Brönsted acid (iii) is formed by dissolving the Brönsted acid (iii) in the same solvent as used for preparing the premixture, and said solution of said Brönsted acid (iii) is added to the premixture. Typically the reaction is started by adding said Brönsted acid (iii) (preferably in the form of a solution in the same solvent as used for preparing the premixture) to said premixture.

In other preferred methods according to the present invention,
(i) said arene-transition metal sandwich complex, and
(ii) said alkyl nitrite
are dissolved in a solution of said Brönsted acid (iii) in a solvent, thus obtaining the reaction mixture.

In specific cases, it is preferred that the Brönsted acid is a non-aqueous Brönsted acid. This is especially the case when the Brönsted acid is a sulfonylimide R¹-SO₂NH-SO₂-R² wherein R¹ and R² are independently selected from the group consisting of CF₃, CHF₂ and CH₂F. Non-aqueous as used herein means that the Brönsted acid does not contain hydrated protons. (The latter is typically the case when a water-soluble acid HX is dissolved in water; HX + H₂O ⇄ H₃O⁺ + X⁻).

In a preferred method according to the present invention,
(i) the arene-transition metal sandwich complex is a metallocene
(ii) the oxidizing agent is selected from the group consisting of alkyl nitrites
(iii) the selected Brönsted acid is a non-aqueous Brönsted acid.

In a more preferred method according to the present invention,
(i) the arene-transition metal sandwich complex is a metallocene
(ii) the oxidizing agent is selected from the group consisting of alkyl nitrites
(iii) the Brönsted acid is a sulfonylimide R¹-SO₂NH-SO₂-R² wherein R¹ and R² are independently selected from the group consisting of CF₃, CHF₂ and CH₂F.

In a further preferred method according to the present invention,
(i) the arene-transition metal sandwich complex is a ferrocene
(ii) the oxidizing agent is selected from the group consisting of alkyl nitrites
(iii) the Brönsted acid is a sulfonylimide R¹-SO₂NH-SO₂-R² wherein R¹ and R² are independently selected from the group consisting of CF₃, CHF₂ and CH₂F.

In a still further preferred method according to the present invention,
(i) the arene-transition metal sandwich complex is a ferrocene
(ii) the oxidizing agent is isoamyl nitrite
(iii) the Brönsted acid is a sulfonylimide R¹-SO₂NH-SO₂-R² wherein R¹ and R² are independently selected from the group consisting of CF₃, CHF₂ and CH₂F.

In said preferred methods using an oxidizing agent selected from the group consisting of alkyl nitrites, the solvent is preferably selected from the group consisting of CH₂Cl₂, CHCl₃ and CCl₄.

In a particularly preferred method according to the present invention,
(i) the arene-transition metal sandwich complex is selected from the group consisting of decamethyl ferrocene, 1,1',3,3',4,4'-hexaisopropyl-2,2',5,5'-tetramethyl ferrocene, 3,3',4,4'-tetraisopropyl-1,1',2,2,5,5'-hexamethyl ferrocene, 1,1'-diethyl-2,2',5,5'-tetramethyl-3,3',4,4'-tetraisopropyl ferrocene, 1,1'-bis(trimethylsilyl)-2,2',5,5'-tetramethyl-3,3',4,4'-tetraisopropyl ferrocene and octaisopropyl ferrocene
(ii) the oxidizing agent is isoamylnitrite
(iii) the Brönsted acid is bis(trifluoromethylsulfonyl)imide (CF₃SO₂)₂NH.
and the solvent is selected from the group consisting of CH₂Cl₂, CHCl₃ and CCl₄.

Said particularly preferred method is a method for preparing a salt selected from the group consisting of decamethyl ferrocenium bis(trifluoromethylsulfonyl)imide, 1,1',3,3',4,4'-hexaisopropyl-2,2',5,5'-tetramethyl ferrocenium bis(trifluoromethylsulfonyl) imide, 3,3',4,4'-tetraisopropyl-1,1',2,2',5,5'-hexamethyl ferrocenium bis(trifluoromethylsulfonyl)imide, 1,1'-diethyl-2,2',5,5'-tetramethyl-3,3',4,4'-tetraisopropyl ferrocenium bis(trifluoromethylsulfonyl) imide, 1,1'-bis(trimethylsilyl)-2,2',5,5'-tetramethyl-3,3',4,4'-tetraisopropyl ferrocenium bis(trifluoromethylsulfonyl)imide and octaisopropyl ferrocenium bis(trifluoromethylsulfonyl)imide.

In the method according to the present invention, the yield of the salt consisting of cations of an arene-transition metal sandwich complex and anions of a Brönsted acid is preferably 80 % or more, more preferably 85 % or more, further preferably 90 % or more and particularly preferably 95 % or more, based on the initial amount of the arene-transition metal sandwich complex.

The chemical composition of the obtained product can be verified by elemental analysis. Details of said analytical technique are known to the skilled person.

The chemical structure of the obtained product can be verified by X-ray diffraction (XRD), nuclear magnetic resonance spectroscopy (NMR) and other suitable analytical techniques. Details of such analytical techniques are known to the skilled person.

In another aspect, the present invention relates to the use of an oxidizing agent selected from the group consisting of alkyl nitrites (ii) in a method as described herein for preparing a salt consisting of cations of an arene-transition metal sandwich complex (i) and anions of a Brönsted acid (iii). Statements made above regarding preferred methods of the present invention apply also to this aspect of the invention.

Preferably, said oxidizing agent is isoamyl nitrite. Especially preferably, isoamyl nitrite is used in a method as described herein for preparing a salt selected from the group consisting of decamethyl ferrocenium bis(trifluoromethylsulfonyl)imide, 1,1',3,3',4,4'-hexaisopropyl-2,2',5,5'-tetramethyl ferrocenium bis(trifluoromethylsulfonyl) imide, 3,3',4,4'-tetraisopropyl-1,1',2,2',5,5'-hexamethyl ferrocenium bis(trifluoromethylsulfonyl)imide, 1,1'-diethyl-2,2',5,5'-tetramethyl-3,3',4,4'-tetraisopropyl ferrocenium bis(trifluoromethylsulfonyl) imide, 1,1'-bis(trimethylsilyl)-2,2',5,5'-tetramethyl-3,3',4,4'-tetraisopropyl ferrocenium bis(trifluoromethylsulfonyl)imide and octaisopropyl ferrocenium bis(trifluoromethylsulfonyl)imide.

The present invention is hereinbelow further illustrated by the following examples.

### Example 1: Preparation of decamethyl ferrocenium bis(trifluoromethylsulfonyl)imide

A premixture was prepared by dissolving
(i) 0.76 g (4.09 mMoles) of decamethyl ferrocene and
(ii) 0.48 g (4.09 mMoles) of isoamyl nitrite
in 15 mL CHCl₃.

A reaction mixture was prepared by adding to said premixture a solution of
(iii) 1.15 g (4.09 mMoles) Bis(trifluormethylsulfonyl)imide
in 15 mL CHCl₃
at room temperature under stirring.

The color of the reaction mixture quickly turned from yellow-orange to dark green upon addition of the solution of (iii), and gas evolved. The reaction mixture was heated under reflux conditions up to its boiling point, and was kept at its boiling point under reflux conditions for one hour.

Then the reaction mixture was allowed to cool and the liquid phase was removed by evaporation so that a solid residue was obtained. The obtained solid residue was digested using either diethyl ether or toluene as the digesting agent. The digesting agent was removed from the digested solid residue by filtration. The digested solid residue was collected and dried at 25 °C for 6 hours in vacuo. The obtained product is a dark green powder.

The amount of the obtained product was 1.7 g, corresponding to a yield of 89.2 % based on the initial amount of decamethyl ferrocene.

The chemical composition of the obtained product was analyzed by elemental analysis. The results are listed in the table below. For comparison, the theoretical weight percentage of each element in decamethyl ferrocenium bis(trifluoromethylsulfonyl)imide are given. The deviations between the calculated and the measured weight percentages are within the accuracy of measurement.

| element | Calculated content/wt.-% | Measured content/wt.-% |
|---|---|---|
| C | 30.9 | 31.2 |
| H | 2.2 | 2.5 |
| N | 3.0 | 4.2 |
| S | 13.8 | 12.6 |
| Fe | 12.0 | 11.5 |

### Example 2: Preparation of 1,1',3,3',4,4'-hexaisopropyl-2,2',5,5'-tetramethyl ferrocenium bis(trifluoromethylsulfonyl)imide

A premixture was prepared by dissolving
(i) 1.00 g (2.02 mMoles) of 1,1',3,3',4,4'-hexaisopropyl-2,2',5,5'-tetramethyl ferrocene and
(ii) 0.237 g (2.02 mMoles) of isoamyl nitrite
in 20 mL CH₂Cl₂.

A reaction mixture was prepared by adding to said premixture a solution of
(iii) 0.568 g (2.02 mMoles) Bis(trifluormethylsulfonyl)imide
in 20 mL CH₂Cl₂
at room temperature under stirring.

The color of the reaction mixture quickly turned from yellow-brown to deep green upon addition of the solution of (iii), and gas evolved. The reaction mixture was kept under stirring at room temperature over night.

Then the liquid phase was removed by evaporation so that a solid residue was obtained. The obtained solid residue was digested five times using diethyl ether as the digesting agent. The digesting agent was removed from the digested solid residue by filtration. The digested solid residue was collected and dried at 25 °C for 6 hours in vacuo. The obtained product is a dark green powder.

The amount of the obtained product was 1.29 g (1.68 mMoles), corresponding to a yield of 83 % based on the initial amount of 1,1',3,3',4,4'-hexaisopropyl-2,2',5,5'-tetramethyl ferrocene.

The chemical composition of the obtained product was analyzed by elemental analysis. The results are listed in the table below. For comparison, the theoretical weight percentage of the corresponding elements in 1,1',3,3',4,4'-hexaisopropyl-2,2',5,5'-tetramethyl ferrocenium bis(trifluoromethylsulfonyl)imide are given. The deviations between the calculated and the measured weight percentages are within the accuracy of measurement.

| element | Calculated content/wt.-% | Measured content/wt.-% |
|---|---|---|
| C | 52.71 | 51.38 |
| H | 7.03 | 7.03 |
| N | 1.81 | 2.09 |
| S | 8.28 | 8.4 |

### Example 3: Preparation of 3,3',4,4'-tetraisopropyl-1,1',2,2',5,5'-hexamethyl ferrocenium bis(trifluoromethylsulfonyl)imide

A reaction mixture was prepared by dissolving
(i) 1.00 g (2.28 mMoles) of 3,3',4,4'-tetraisopropyl-1,1',2,2',5,5'-hexamethyl ferrocene and
(ii) 0.27 g (2.30 mMoles) of isoamyl nitrite
   in a stirred solution of
(iii) 0.64 g (2.28 mMoles) Bis(trifluormethylsulfonyl)imide
in 20 mL CH₂Cl₂
at room temperature under stirring.

The color of the reaction mixture quickly turned from yellow to deep green upon addition of the solution of (iii), and gas evolved. The reaction mixture was kept under stirring at room temperature over 16 hours.

Then the liquid phase was removed by evaporation so that a solid residue was obtained. The obtained solid residue was digested using diethyl ether as the digesting agent. The digesting agent was removed from the digested solid residue by filtration. The digested solid residue was collected and dried at 25 °C for 6 hours in vacuo. The obtained product is a dark green powder.

The amount of the obtained product was 1.57 g (2.17 mMoles), corresponding to a yield of 95 % based on the initial amount of 3,3',4,4'-tetraisopropyl-1,1',2,2',5,5'-hexamethyl ferrocene.

The chemical composition of the obtained product was analyzed by elemental analysis. The results are listed in the table below. For comparison, the theoretical weight percentage of the corresponding elements in 3,3',4,4'-tetraisopropyl-1,1',2,2',5,5'-hexamethyl ferrocenium bis(trifluoromethylsulfonyl)imide are given. The deviations between the calculated and the measured weight percentages are within the accuracy of measurement.

| element | Calculated content/wt.-% | Measured content/wt.-% |
|---|---|---|
| C | 50.14 | 49.86 |
| H | 6.45 | 6.44 |
| N | 1.95 | 1.89 |
| S | 8.92 | 9.03 |

### Example 4: Preparation of 1,1'-diethyl-2,2',5,5'-tetramethyl-3,3',4,4'-tetraisopropyl ferrocenium bis(trifluoromethylsulfonyl)imide

A reaction mixture was prepared by dissolving
(i) 1.00 g (2.14 mMoles) of 1,1'-diethyl-2,2',5,5'-tetramethyl-3,3',4,4'-tetraisopropyl ferrocene
   in 20 ml CH₂Cl₂ and adding
(ii) 0.25 g (2.14 mMoles) of isoamyl nitrite.

A reaction mixture was prepared by adding to said premixture a solution of
(iii) 0.602 g (2.14 mMoles) Bis(trifluormethylsulfonyl)imide
in 20 mL CH₂Cl₂
at room temperature under stirring.

The color of the reaction mixture quickly turned from yellow to deep green upon addition of the solution of (iii), and gas evolved. The reaction mixture was kept under stirring at room temperature over 18 hours.

Then the liquid phase was removed by evaporation so that a solid residue was obtained. The obtained solid residue was digested using diethyl ether as the digesting agent. The digesting agent was removed from the digested solid residue by filtration. The digested solid residue was collected and dried at 25 °C for 6 hours in vacuo. The obtained product is a dark green powder.

The amount of the obtained product was 1.45 g (1.94 mMoles), corresponding to a yield of 91 % based on the initial amount of 1,1'-diethyl-2,2',5,5'-tetramethyl-3,3',4,4'-tetraisopropyl ferrocene.

The chemical composition of the obtained product was analyzed by elemental analysis. The results are listed in the table below. For comparison, the theoretical weight percentage of the corresponding elements in 1,1'-diethyl-2,2',5,5'-tetramethyl-3,3',4,4'-tetraisopropyl ferrocenium bis(trifluoromethylsulfonyl)imide are given. The deviations between the calculated and the measured weight percentages are within the accuracy of measurement.

| element | Calculated content/wt.-% | Measured content/wt.-% |
|---|---|---|
| C | 50.74 | 50.87 |
| H | 6.75 | 6.54 |
| N | 1.88 | 1.92 |
| S | 8.59 | 8.66 |

### Example 5: Preparation of bis(trimethylsilyl)-2,2',5,5'-tetramethyl-3,3',4,4'-tetraisopropyl ferrocenium bis(trifluoromethylsulfonyl)imide

A reaction mixture was prepared by dissolving
(i) 1.00 g (1.8 mMoles) of bis(trimethylsilyl)-2,2',5,5'-tetramethyl-3,3',4,4'-tetraisopropyl ferrocene
   in 20 ml CH₂Cl₂ and adding
(ii) 0.211 g (1.8 mMoles) of isoamyl nitrite.

A reaction mixture was prepared by adding to said premixture a solution of
(iii) 0.507 g (1.8 mMoles) Bis(trifluormethylsulfonyl)imide
in 20 mL CH₂Cl₂
at room temperature under stirring.

The color of the reaction mixture quickly turned from orange to deep green upon addition of the solution of (iii), and gas evolved. The reaction mixture was kept under stirring at room temperature over 18 hours.

Then the liquid phase was removed by evaporation so that a solid residue was obtained. The obtained solid residue was digested four times using diethyl ether as the digesting agent. The digesting agent was removed from the digested solid residue by filtration. The digested solid residue was collected and dried at 25 °C for 6 hours in vacuo. The obtained product is a dark green powder.

The amount of the obtained product was 1.4 g (1.68 mMoles), corresponding to a yield of 93 % based on the initial amount of bis(trimethylsilyl)-2,2',5,5'-tetramethyl-3,3',4,4'-tetraisopropyl ferrocene.

The chemical composition of the obtained product was analyzed by elemental analysis. The results are listed in the table below. For comparison, the theoretical weight percentage of the corresponding elements in bis(trimethylsilyl)-2,2',5,5'-tetramethyl-3,3',4,4'-tetraisopropyl bis(trifluoromethylsulfonyl)imide are given. The deviations between the calculated and the measured weight percentages are within the accuracy of measurement.

| element | Calculated content/wt.-% | Measured content/wt.-% |
|---|---|---|
| C | 48.91 | 48.86 |
| H | 7.00 | 6.35 |
| N | 1.68 | 1.84 |
| S | 7.58 | 7.51 |

### Example 6: Preparation of octaisopropyl ferrocenium bis(trifluoromethylsulfonyl)imide

A premixture was prepared by dissolving
(i) 0.80 g (1.53 mMoles) of octaisopropyl ferrocene and
(ii) 0.179 g (1.53 mMoles) of isoamyl nitrite
in 30 mL CH₂Cl₂.

A reaction mixture was prepared by adding to said premixture a solution of
(iii) 0.430 g (1.53 mMoles) Bis(trifluormethylsulfonyl)imide
in 150 mL CH₂Cl₂
at room temperature under stirring.

The color of the reaction mixture quickly turned from salmon to deep green upon addition of the solution of (iii), and gas evolved. The reaction mixture was kept under stirring at room temperature over night.

Then the liquid phase was removed by evaporation so that a solid residue was obtained. The obtained solid residue was digested using diethyl ether as the digesting agent. The digesting agent was removed from the digested solid residue by filtration. The digested solid residue was collected and dried at 25 °C for 6 hours in vacuo. The obtained product is a dark green powder.

The amount of the obtained product was 1.16 g (1.44 mMoles), corresponding to a yield of 94 % based on the initial amount of octaisopropyl ferrocene.

The chemical composition of the obtained product was analyzed by elemental analysis. The results are listed in the table below. For comparison, the theoretical weight percentage of the corresponding elements in octaisopropyl ferrocenium bis(trifluoromethylsulfonyl)imide are given. The deviations between the calculated and the measured weight percentages are within the accuracy of measurement.

| element | Calculated content/wt.-% | Measured content/wt.-% |
|---|---|---|
| C | 53.86 | 53.9 |
| H | 7.28 | 6.64 |
| N | 1.74 | 1.74 |
| S | 7.99 | 7.86 |

## Claims

1. Method for preparing a salt consisting of cations of an arene-transition metal sandwich complex (i) selected from the group consisting of bis-benzenechromium and metallocenes and anions of a BrOnsted acid (iii) selected from the group consisting of sulfonylimides R¹⁻SO₂₋NH-SO₂-R² wherein R¹ and R² are independently selected from the group consisting of CF₃, CHF₂ and CH₂F, fluoroboric acid HBF₄, hexafluorophosphoric acid HPF₆. and trifluoroacetic acid, fluorinated alcohols and fluorinated phenols
said method comprising
reacting a reaction mixture comprising, dissolved in a solvent, the reactants
(i) an arene-transition metal sandwich complex is selected from the group consisting of bis-benzenechromium and metallocenes
(ii) an oxidizing agent selected from the group consisting of alkyl nitrites
(iii) a Brönsted acid selected from the group consisting of sulfonylimides R¹-SO₂-NH-SO₂-R² wherein R¹ and R² are independently selected from the group consisting of CF₃, CHF₂ and CH₂F, fluoroboric acid HBF₄, hexafluorophosphoric acid HPF₆, trifluoroacetic acid, fluorinated alcohols and fluorinated phenols.

2. Method according to claim 1, wherein said reaction mixture is prepared by dissolving in a solvent
(i) an arene-transition metal sandwich complex as defined in claim 1
(ii) an oxidizing agent selected from the group consisting of alkyl nitrites
(iii) a Bronsted acid as defined in claim 1.

3. Method according to claim 2, wherein
(i) said arene-transition metal sandwich complex and
(ii) said oxidizing agent
are dissolved in the solvent in a molar ratio (i)/(ii) in the range of from 08:1 to 1:0.8.

4. Method according to claim 1 or 2, wherein
said arene-transition metal sandwich complex (i) is a metallocene selected from the group consisting of ferrocenes, nickelocenes, cobaltocenes and osmocenes.

5. Method according to claim 4, wherein
said arene-transition metal sandwich complex (i) is selected from the group consisting of decamethyl ferrocene, 1,1',3,3',4,4'-hexaisopropyl-2,2',5,5'-tetramethyl ferrocene, 3,3',4,4'-tetraisopropyl-1,1',2,2',5,5'-hexamethyl ferrocene, 1,1'-diethyl-2.2',5,5'-tetramethyl-3,3',4,4'-tetraisopropyl ferrocene, 1,1'-bis(trimethylsilyl)-2,2',5,5'-tetramethyl-3,3',4,4'-tetraisopropyl ferrocene and octaisopropyl ferrocene.

6. Method according to any preceding claim, wherein
said oxidizing agent (ii) is isoamyl nitrite.

7. Method according to any preceding claim, wherein
said Brönsted acid (iii) is a sulfonylimide R¹-SO₂-NH-SO₂-R² wherein R¹ and R² are independently selected from the group consisting of CF₃, CHF₂ and CH₂F.

8. Method according to any preceding claim, wherein
said solvent is selected from the group consisting of chlorinated hydrocarbons, toluene, diethylether, methyl-tert-butyl-ether, tetrahydrofurane and esters of carboxylic acids.

9. Method according to any preceding claim, wherein
reacting the reaction mixture comprises keeping the reaction mixture at its boiling point under reflux conditions, preferably for a duration of at most two hours,

10. Method according to any preceding claim, further comprising the steps of
- evaporating the solvent from the reaction mixture to obtain a solid residue
- digesting the obtained solid residue with a digesting agent
- removing the digesting agent from the digested solid residue
- drying the digested solid residue.

11. Method according to claim 10, wherein
said digesting agent is selected from the group consisting of chlorinated hydrocarbons, toluene, diethylether, methyl-tert-butyl-ether, tetrahydrofurane and esters of carboxylic acids.

12. Method according to any preceding claim, wherein
(i) the arene-transition metal sandwich complex is a ferrocene
(ii) the oxidizing agent is isoamyl nitrite
(iii) the Brönsted acid is a sulfonylimide R¹-SO₂-NH-SO₂₋R² wherein R¹ and R² are independently selected from the group consisting of CF₃, CHF₂ and CH₂F.

13. Method according to any preceding claim, wherein
(i) the metallocene is selected from the group consisting of decamethyl ferrocene, 1,1',3,3',4,4'-hexaisopropyl-2,2',5,5'-tetramethyl ferrocene, 3,3',4,4'-tetraisopropyl-1,1',2,2',5,5'-hexamethyl ferrocene, 1,1'-diethyl-2,2',5,5'-tetramethyl-3,3',4,4'-tetraisopropyl ferrocene, 1,1'-bis(trimethylsilyl)-2,2',5,5'-tetramethyl-3,3',4,4'-tetraisopropyl ferrocene and octaisopropyl ferrocene
(ii) the oxidizing agent is isoamylnitrite
(iii) the Brönsted acid is bis(trifluoromethylsulfonyl)imide and the solvent is selected from the group consisting of CH₂Cl₂, CHCl₃ and CCl₄.

14. Use of an oxidizing agent selected from the group consisting of alkyl nitrites (ii) in a method according to any of claims 1 to 13 for preparing a salt consisting of cations of an arene-transition metal sandwich complex (i) selected from the group consisting of bis-benzenechromium and metallocenes and anions of a Brönsted acid (iii) selected from the group consisting of sulfonylimides R¹-SO₂NH-SO₂-R² wherein R¹ and R² are independently selected from the group consisting of CF₃, CHF₂ and CH₂F, fluoroboric acid HBF₄, hexafluorophosphoric acid HPF₆, trifluoroacetic acid, fluorinated alcohols and fluorinated phenols.

15. Use according to claim 14, wherein said oxidizing agent is isoamyl nitrite.

## Patentansprüche

1. Verfahren zur Herstellung eines Salzes aus Kationen eines Aren-Übergangsmetall-Sandwichkomplexes (i) aus der Gruppe bestehend aus Bisbenzolchrom und Metallocenen und Anionen einer Brönsted-Säure (iii) aus der Gruppe bestehend aus Sulfonylimiden R¹-SO₂-NH-SO₂-R², wobei R¹ und R² unabhängig aus der Gruppe bestehend aus CF₃, CHF₂ und CH₂F ausgewählt sind, Fluoroborsäure HBF₄, Hexafluorophosphorsäure HPF₆ und Trifluoressigsäure, fluorierten Alkoholen und fluorierten Phenolen,
wobei das Verfahren Folgendes umfasst:
Umsetzen einer Reaktionsmischung, die die folgenden Reaktanten in einem Lösungsmittel gelöst umfasst:
(i) einen Aren-Übergangsmetall-Sandwichkomplex aus der Gruppe bestehend aus Bisbenzolchrom und Metallocenen,
(ii) ein Oxidationsmittel aus der Gruppe bestehend aus Alkylnitriten,
(iii) eine Brönsted-Säure aus der Gruppe bestehend aus Sulfonylimiden R¹-SO₂-NH-SO₂-R², wobei R¹ und R² unabhängig aus der Gruppe bestehend aus CF₃, CHF₂ und CH₂F ausgewählt sind, Fluoroborsäure HBF₄, Hexafluorophosphorsäure HPF₆, Trifluoressigsäure, fluorierten Alkoholen und fluorierten Phenolen.

2. Verfahren nach Anspruch 1, bei dem die Reaktionsmischung durch Lösen von
(i) einem Aren-Übergangsmetall-Sandwichkomplex gemäß Anspruch 1,
(ii) einem Oxidationsmittel aus der Gruppe bestehend aus Alkylnitriten,
(iii) einer Brönsted-Säure gemäß Anspruch 1 in einem Lösungsmittel hergestellt wird.

3. Verfahren nach Anspruch 2, bei dem
(i) der Aren-Übergangsmetall-Sandwichkomplex und
(ii) das Oxidationsmittel
in dem Lösungsmittel in einem Molverhältnis (i)/(ii) im Bereich von 0,8:1 bis 1:0,8 gelöst werden.

4. Verfahren nach Anspruch 1 oder 2, bei dem
es sich bei dem Aren-Übergangsmetall-Sandwichkomplex (i) um ein Metallocen aus der Gruppe bestehend aus Ferrocenen, Nickelocenen, Cobaltocenen und Osmocenen handelt.

5. Verfahren nach Anspruch 4, bei dem
der Aren-Übergangsmetall-Sandwichkomplex (i) aus der Gruppe bestehend aus Decamethylferrocen, 1,1',3,3',4,4'-Hexaisopropyl-2,2',5,5'-tetramethylferrocen, 3,3',4,4'-Tetraisopropyl-1,1',2,2',5,5'-hexamethylferrocen, 1,1'-Diethyl-2,2',5,5'-tetramethyl-3,3',4,4'-tetraisopropyl-ferrocen, 1,1'-Bis(trimethylsilyl)-2,2',5,5'-tetramethyl-3,3',4,4'-tetraisopropylferrocen und Octaisopropylferrocen ausgewählt wird.

6. Verfahren nach einem der vorhergehenden Ansprüche, bei dem
es sich bei dem Oxidationsmittel (ii) um Isoamylnitrit handelt.

7. Verfahren nach einem der vorhergehenden Ansprüche, bei dem
es sich bei der Brönsted-Säure (iii) um ein Sulfonylimid R¹-SO₂-NH-SO₂-R², wobei R¹ und R² unabhängig aus der Gruppe bestehend aus CF₃, CHF₂ und CH₂F ausgewählt sind, handelt.

8. Verfahren nach einem der vorhergehenden Ansprüche, bei dem
das Lösungsmittel aus der Gruppe bestehend aus chlorierten Kohlenwasserstoffen, Toluol, Diethylether, Methyl-tert-butylether, Tetrahydrofuran und Estern von Carbonsäuren ausgewählt wird.

9. Verfahren nach einem der vorhergehenden Ansprüche, bei dem
das Umsetzen der Reaktionsmischung das Halten der Reaktionsmischung an ihrem Siedepunkt unter Rückflussbedingungen, vorzugsweise für eine Dauer von höchstens zwei Stunden, umfasst.

10. Verfahren nach einem der vorhergehenden Ansprüche, bei dem man ferner
- das Lösungsmittel von der Reaktionsmischung abdampft, was einen festen Rückstand ergibt,
- den erhaltenen festen Rückstand mit einem Digeriermittel digeriert,
- das Digeriermittel von dem digerierten festen Rückstand entfernt,
- den digerierten festen Rückstand trocknet.

11. Verfahren nach Anspruch 10, bei dem
das Digeriermittel aus der Gruppe bestehend aus chlorierten Kohlenwasserstoffen, Toluol, Diethylether, Methyl-tert-butylether, Tetrahydrofuran und Estern von Carbonsäuren ausgewählt wird.

12. Verfahren nach einem der vorhergehenden Ansprüche, bei dem
(i) es sich bei dem Aren-Übergangsmetall-Sandwichkomplex um ein Ferrocen handelt,
(ii) es sich bei dem Oxidationsmittel um Isoamylnitrit handelt,
(iii) es sich bei der Brönsted-Säure um ein Sulfonylimid R¹-SO₂-NH-SO₂-R², wobei R¹ und R² unabhängig aus der Gruppe bestehend aus CF₃, CHF₂ und CH₂F ausgewählt sind, handelt.

13. Verfahren nach einem der vorhergehenden Ansprüche, bei dem
(i) das Metallocen aus der Gruppe bestehend aus Decamethylferrocen, 1,1',3,3',4,4'-Hexaisopropyl-2,2',5,5'-tetramethylferrocen, 3,3',4,4'-Tetraisopropyl-1,1',2,2',5,5'-hexamethylferrocen, 1,1'-Diethyl-2,2',5,5'-tetramethyl-3,3',4,4'-tetraisopropyl-ferrocen, 1,1'-Bis(trimethylsilyl)-2,2',5,5'-tetramethyl-3,3',4,4'-tetraiso-propylferrocen und Octaisopropylferrocen ausgewählt wird,
(ii) es sich bei dem Oxidationsmittel um Isoamylnitrit handelt,
(iii) es sich bei der Brönsted-Säure um Bis-(trifluormethylsulfonyl)imid handelt
und das Lösungsmittel aus der Gruppe bestehend aus CH₂Cl₂, CHCl₃ und CCl₄ ausgewählt wird.

14. Verwendung eines Oxidationsmittels aus der Gruppe bestehend aus Alkylnitriten (ii) bei einem Verfahren nach einem der Ansprüche 1 bis 13 zur Herstellung eines Salzes aus Kationen eines Aren-Übergangsmetall-Sandwichkomplexes (i) aus der Gruppe bestehend aus Bisbenzolchrom und Metallocenen und Anionen einer Brönsted-Säure (iii) aus der Gruppe bestehend aus Sulfonylimiden R¹-SO₂-NH-SO₂-R², wobei R¹ und R² unabhängig aus der Gruppe bestehend aus CF₃, CHF₂ und CH₂F ausgewählt sind, Fluoroborsäure HBF₄, Hexafluorophosphorsäure HPF₆, Trifluoressigsäure, fluorierten Alkoholen und fluorierten Phenolen.

15. Verwendung nach Anspruch 14, wobei es sich bei dem Oxidationsmittel um Isoamylnitrit handelt.

## Revendications

1. Procédé pour la préparation d'un sel constitué de cations d'un complexe sandwich arène-métal de transition (i) choisi dans le groupe constitué par le bis-benzènechrome et les métallocènes et d'anions d'un acide de Brönsted (iii) choisi dans le groupe constitué par les sulfonylimides R¹-SO₂-NH-SO₂-R², R¹ et R² étant indépendamment choisis dans le groupe constitué par CF₃, CHF₂ et CH₂F, l'acide fluoroborique HBF₄, l'acide hexafluorophosphorique HPF₆ et l'acide trifluoroacétique, les alcools fluorés et les phénols fluorés
ledit procédé comprenant
la mise en réaction d'un mélange réactionnel comprenant, dissouts dans un solvant, les réactants
(i) un complexe sandwich arène-métal de transition choisi dans le groupe constitué par le bis-benzènechrome et les métallocènes
(ii) un agent d'oxydation choisi dans le groupe constitué par les nitrites d'alkyle
(iii) un acide de Brönsted choisi dans le groupe constitué par les sulfonylimides R¹-SO₂-NH-SO₂-R², R¹ et R² étant indépendamment choisis dans le groupe constitué par CF₃, CHF₂ et CH₂F, l'acide fluoroborique HBF₄, l'acide hexafluorophosphorique HPF₆ et l'acide trifluoroacétique, les alcools fluorés et les phénols fluorés.

2. Procédé selon la revendication 1, ledit mélange réactionnel étant préparé en dissolvant dans un solvant
(i) un complexe sandwich arène-métal de transition tel que défini selon la revendication 1
(ii) un agent d'oxydation choisi dans le groupe constitué par les nitrites d'alkyle
(iii) un acide de Brönsted tel que défini selon la revendication 1.

3. Procédé selon la revendication 2,
(i) ledit complexe sandwich arène-métal de transition et
(ii) ledit agent d'oxydation
étant dissouts dans le solvant en un rapport molaire (i)/(ii) dans la plage de 0,8:1 à 1:0,8.

4. Procédé selon la revendication 1 ou 2,
ledit complexe sandwich arène-métal de transition (i) étant un métallocène choisi dans le groupe constitué par les ferrocènes, les nickelocènes, les cobaltocènes et les osmocènes.

5. Procédé selon la revendication 4,
ledit complexe sandwich arène-métal de transition (i) étant choisi dans le groupe constitué par le décaméthylferrocène, le 1,1',3,3',4,4'-hexaisopropyl-2,2',5,5'-tétraméthyferrocène, le 3,3',4,4'-tétraisopropyl-1,1',2,2',5,5'-hexaméthyferrocène, le 1,1'-diéthyl-2, 2',5,5'-tétraméthyl-3,3',4,4'-tétraisopropylferrocène, le 1,1'-bis(triméthylsilyl)-2,2',5,5'-tétraméthyl-3,3',4,4'-tétraisopropylferrocène et l'octaisopropylferrocène.

6. Procédé selon l'une quelconque des revendications précédentes,
ledit agent d'oxydation (ii) étant le nitrite d'isoamyle.

7. Procédé selon l'une quelconque des revendications précédentes,
ledit acide de Brönsted (iii) étant un sulfonylimide R¹-SO₂-NH-SO₂-R², R¹ et R² étant indépendamment choisis dans le groupe constitué par CF₃, CHF₂ et CH₂F.

8. Procédé selon l'une quelconque des revendications précédentes,
ledit solvant étant choisi dans le groupe constitué par les hydrocarbures chlorés, le toluène, l'éther diéthylique, l'éther de méthyle et de tert-butyle, le tétrahydrofuranne et les esters d'acides carboxyliques.

9. Procédé selon l'une quelconque des revendications précédentes,
la mise en réaction du mélange réactionnel comprenant le fait de laisser le mélange réactionnel à son point d'ébullition dans des conditions de reflux, préférablement pendant une durée d'au plus deux heures.

10. Procédé selon l'une quelconque des revendications précédentes, comprenant en outre les étapes de
- évaporation du solvant du mélange réactionnel pour obtenir un résidu solide
- digestion du résidu solide obtenu avec un agent de digestion
- élimination de l'agent de digestion du résidu solide digéré
- séchage du résidu solide digéré.

11. Procédé selon la revendication 10,
ledit agent de digestion étant choisi dans le groupe constitué par les hydrocarbures chlorés, le toluène, l'éther diéthylique, l'éther de méthyle et de tert-butyle, le tétrahydrofuranne et les esters d'acides carboxyliques.

12. Procédé selon l'une quelconque des revendications précédentes,
(i) le complexe sandwich arène-métal de transition étant un ferrocène
(ii) l'agent d'oxydation étant le nitrite d'isoamyle
(iii) l'acide de Brönsted étant un sulfonylimide R¹-SO₂-NH-SO₂-R², R¹ et R² étant indépendamment choisis dans le groupe constitué par CF₃, CHF₂ et CH₂F.

13. Procédé selon l'une quelconque des revendications précédentes,
(i) le métallocène étant choisi dans le groupe constitué par le décaméthylferrocène, le 1,1',3,3',4,4'-hexaisopropyl-2,2',5,5'-tétraméthyferrocène, le 3,3',4,4'-tétraisopropyl-1,1',2,2',5,5'-hexaméthyferrocène, le 1,1'-diéthyl-2,2',5,5'-tétraméthyl-3,3',4,4'-tétraisopropylferrocène, le 1,1'-bis(triméthylsilyl)-2,2',5,5'-tétraméthyl-3,3',4,4'-tétraisopropylferrocène et l'octaisopropylferrocène
(ii) l'agent d'oxydation étant le nitrite d'isoamyle
(iii) l'acide de Brönsted étant le bis(trifluorométhylsulfonyl)imide
et le solvant étant choisi dans le groupe constitué par CH₂Cl₂, CHCl₃ et CCl₄.

14. Utilisation d'un agent oxydant choisi dans le groupe constitué par les nitrites d'alkyle (ii) dans un procédé selon l'une quelconque des revendications 1 à 13 pour la préparation d'un sel constitué de cations d'un complexe sandwich arène-métal de transition (i) choisi dans le groupe constitué par le bis-benzènechrome et les métallocènes et d'anions d'un acide de Brönsted (iii) choisi dans le groupe constitué par les sulfonylimides R¹-SO₂-NH-SO₂-R², R¹ et R² étant indépendamment choisis dans le groupe constitué par CF₃, CHF₂ et CH₂F, l'acide fluoroborique HBF₄, l'acide hexafluorophosphorique HPF₆ et l'acide trifluoroacétique, les alcools fluorés et les phénols fluorés.

15. Utilisation selon la revendication 14, ledit agent d'oxydation étant le nitrite d'isoamyle.
